# EUROPÄISCHE PATENTSCHRIFT

(11) **EP 4 346 539 B1**
(45) Veröffentlichungstag und Bekanntmachung des Hinweises auf die Patenterteilung: **09.04.2025**
(21) Anmeldenummer: 22725276.4
(22) Anmeldetag: 19.05.2022
(51) Int. Cl.: A61B 1/00

(54) **OPTISCHES INSTRUMENT**
OPTICAL INSTRUMENT
INSTRUMENT OPTIQUE

(30) Priorität: 26.05.2021 DE 102021113615
(43) Veröffentlichungstag der Anmeldung: 10.04.2024
(73) Patentinhaber: Karl Storz SE & Co. KG, 78532 Tuttlingen (DE)
(72) Erfinder: FORSTER, Jonas, 78532 Tuttlingen (DE); HENI, Andreas, 78532 Tuttlingen (DE); HENI, Pascal, 78532 Tuttlingen (DE); KUPFERSCHMID, Markus, 78532 Tuttlingen (DE); ULMSCHNEIDER, Daniel, 78532 Tuttlingen (DE)
(86) Internationale Anmeldenummer: PCT/EP2022/063506
(87) Internationale Veröffentlichungsnummer: WO 2022/248320

(56) Entgegenhaltungen:
- DE-A1- 3 920 494
- JP-A- H10 165 367
- US-A1- 2017 280 037
- US-A1- 2018 317 758

## Beschreibung

Die Erfindung betrifft ein optisches Instrument, insbesondere ein Endoskop oder ein Exoskop.

Optische Instrumente, beispielhaft Endoskope, weisen in der Regel ein integriertes Beleuchtungssystem auf, insbesondere eine LED oder mehrere LEDs in einem Gehäuse des Endoskops. Alternativ sind Endoskope bekannt, die eine externe Lichtzuführung bzw. einen externen Lichtanschluss aufweisen. Auf den Lichtanschluss kann mittels eines Lichtkabels von einer externen Lichtquelle Licht in das Endoskop geleitet und dort mittels Lichtfasern an das distale Ende des Endoskops weitergeleitet werden. So offenbart das Dokument US 2018/317758 A1 ein Endoskop dessen Lichtanschluss mittels eines Lichtkabels zwischen zwei externen Lichtquellen wechseln kann.

Es ist eine Aufgabe er vorliegenden Erfindung ein verbessertes optisches Instrument aufzuzeigen, welches eine höhere Flexibilität bei den Beleuchtungsmöglichkeiten bietet.

Die Aufgabe wird gelöst durch ein optisches Instrument mit einem Gehäuse, wobei in dem Gehäuse eine Lichtquelle angeordnet ist, die dafür ausgebildet ist, einen ersten Lichtstrom zu erzeugen, der an einer ersten Grenzfläche innerhalb des Gehäuses austritt, und eine Lichtzuführung von außerhalb des Gehäuses durch eine Gehäusewand des Gehäuses geführt ist, die dafür ausgebildet ist, einen zweiten Lichtstrom von außerhalb des Gehäuses in ein Inneres des Gehäuses zu führen und an einer zweiten Grenzfläche innerhalb des Gehäuses austreten zu lassen, wobei in dem Gehäuse eine Lichtführung angeordnet ist, die dafür ausgebildet ist, Licht an einem ersten Ende über eine dritte Grenzfläche aufzunehmen und an einem zweiten Ende über eine vierte Grenzfläche an einem distalen Ende des optischen Instruments austreten zu lassen, wobei die dritte Grenzfläche relativ zu der ersten und der zweiten Grenzfläche derart verlagerbar angeordnet ist, dass die dritte Grenzfläche in einer ersten Position Licht von der ersten Grenzfläche aufnehmen kann und in einer zweiten Position Licht von der zweiten Grenzfläche aufnehmen kann.

Auf diese Weise wird es ermöglicht, mindestens zwei unterschiedliche Beleuchtungssysteme in einem optischen Instrument zu nutzen, welche wechselweise genutzt werden können. Es ist auch möglich das jeweilige Beleuchtungssystem in Abhängigkeit von der Position der dritten Grenzfläche ein- bzw. auszuschalten.

Dabei kann die Beleuchtung entweder über eine externe Lichtquelle erfolgen, die über die Lichtzuführung, insbesondere ein Lichtkabel, mit dem optischen Instrument verbunden ist. Alternativ erfolgt die Beleuchtung erfolgt über die integrierte Lichtquelle, insbesondere eine LED oder mehrere LEDs. Dabei kann die Energieversorgung der Lichtquelle insbesondere über das bei Videoendoskopen üblicherweise vorhandene Datenkabel erfolgen.

Für den Wechsel zwischen der ersten und zweiten Position ist im optischen Instrument eine Wechselvorrichtung angebracht, mit der der im optischen Instrument befindliche Lichtleiter hin- und hergeschaltet werden kann, so dass dieser entweder mit Licht der intern im optischen Instrument vorhandenen Lichtquelle optisch gekoppelt ist wird oder durch den vorhandenen Lichtanschluss von einer externen Lichtquelle versorgt wird. So kann der Anwender mit ein und demselben Gerät zwischen verschiedenen Beleuchtungssystemen wechseln, wodurch sich ein hohes Maß an Flexibilität ergibt.

Je nach der Anforderung des Anwenders kann bei dem Beleuchtungsmodus mittels der internen Lichtquelle auf ein an das optische Instrument angeschlossenes Lichtkabel verzichtet werden. Benötigt der Anwender eine höhere Lichtintensität oder ein Licht, z.B. einer bestimmten Wellenlänge, das nicht von der internen Lichtquelle bereitgestellt werden kann, so wird mittels der Lichtzuführung, insbesondere ein Lichtkabel, eine externe Lichtquelle an das optische Instrument angeschlossen. Dabei kann z.B. mittels eines mechanischen, magnetischen oder elektrischen Schalters zwischen den verschiedenen Lichtquellen geschaltet werden.

Die interne Lichtquelle kann im Schaft oder im Handgriff des optischen Instruments angeordnet werden, wobei diese jeweils ein separater, koppelbarer Teil des optischen Instruments sein können. Auch die Lichtzuführung, insbesondere ein Lichtanschluss, für die externe Lichtquelle kann im Schaft oder im Handgriff platziert werden kann, wobei diese wiederum jeweils ein separater, koppelbarer Teil des optischen Instruments sein können.

Dadurch ist die Aufgabe vollständig gelöst.

Bei einer bevorzugten Ausgestaltung ist das erste Ende der Lichtführung in einer Führung linear verlagerbar gehalten.

Diese Ausgestaltung ermöglicht dem ersten Ende der Lichtführung eine gewisse Verlagerung, wenn die dritte Grenzfläche von der ersten in die zweite Position, und umgekehrt, verlagert wird. Es ist in aller Regel erforderlich, dass die Lichtführung im Gehäuse des optischen Instruments an einer definierten Stelle bzw. Fixpunkt gehalten wird. Bei der Verlagerung von der ersten in die zweite Position, und umgekehrt, kann sich der Verlauf der Lichtführung vom Fixpunkt in Richtung des ersten Endes verändern. Die lineare Verlagerbarkeit bietet den Freiheitsgrad diese Änderung ohne eine mechanische Belastung der Lichtführung aufzufangen.

Bei einer weiteren bevorzugten Ausgestaltung weist das erste Ende eine Hülse auf und ist die Hülse in der Führung linear verlagerbar gehalten.

Diese Ausgestaltung ermöglicht eine gute Verlagerbarkeit des ersten Endes.

Bei einer weiteren bevorzugten Ausgestaltung sind die Führung und die Hülse zylinderförmig ausgebildet.

Diese Ausgestaltung bietet zusätzlich einen weiteren Freiheitsgrad, da nun das erste Ende innerhalb der Führung rotieren kann.

Bei einer weiteren bevorzugten Ausgestaltung weist die Hülse einen Flansch auf, der einen Anschlag für eine Verlagerung der Hülse bildet.

Diese Ausgestaltung ermöglicht eine definierte Verlagerung der Hülse. Dabei kann der Anschlag eine Verlagerung in proximaler oder distaler Richtung begrenzen. Werden zwei Anschläge an der Hülse verwendet kann eine Verlagerung in proximaler und distaler Richtung begrenzt werden. Zudem hat die Hülse, und damit das erste Ende, am Anschlag eine definierte Position.

Bei einer weiteren bevorzugten Ausgestaltung ist die Lichtquelle über eine Energiezuführung, die von außerhalb des Gehäuses durch die Gehäusewand des Gehäuses geführt ist, mit Energie versorgt.

Diese Ausgestaltung ermöglicht eine zuverlässige Energieversorgung der internen Lichtquelle. Ist eine Energiezuführung bereits vorhanden, zum Beispiel in einer Spannungsversorgung für einen Bildsensor im optischen Instrument, kann diese bestehende Energiezuführung auch zur Energieversorgung der internen Lichtquelle genutzt werden.

Bei einer weiteren bevorzugten Ausgestaltung ist das erste Ende auf einem inneren Ring innerhalb des Gehäuses angeordnet, der um eine Längsachse des optischen Instruments rotierbar angeordnet ist.

Diese Ausgestaltung ermöglicht eine geführte Verlagerung des ersten Endes.

Bei einer weiteren bevorzugten Ausgestaltung weist das optische Instrument ferner einen äußeren Ring auf, der außerhalb des Gehäuses angeordnet ist und um die Längsachse des optischen Instruments rotierbar angeordnet ist, wobei am inneren Ring mindestens ein erster Magnet angeordnet ist, am äußeren Ring mindestens ein zweiter Magnet angeordnet ist und der erste Magnet und der zweite Magnet derart zusammenwirken, dass mittels einer Rotation des äußeren Rings eine Rotation des inneren Rings bewirkt werden kann.

Diese Ausgestaltung ermöglicht eine Verlagerung des inneren Rings durch eine Betätigung von außen, ohne dafür eine Öffnung des Gehäuses bereitstellen zu müssen. Es stehen sich dabei insbesondere erste und zweite Magnete mit unterschiedlichen Polen gegenüber, so dass die ersten und zweiten Magnete derart zusammenwirken, dass ein Abstand zwischen den ersten und zweiten Magneten möglichst gering ist.

Bei einer weiteren bevorzugten Ausgestaltung weist das Gehäuse zwei nach innen gerichtete erste Anschläge auf, die mit einem ersten Vorsprung am inneren Ring zusammenwirken und eine Verlagerung des inneren Rings auf einen ersten Winkelbereich begrenzen, und weist das Gehäuse zwei nach außen gerichtete zweite Anschläge auf, die mit einem zweiten Vorsprung am äußeren Ring zusammenwirken und eine Verlagerung des äußeren Rings auf einen zweiten Winkelbereich begrenzen, wobei der zweite Winkelbereich größer als der erste Winkelbereich ist.

Diese Ausgestaltung ermöglicht es, den Vorsprung des inneren Rings mit einer gewissen Kraft gegen einen der ersten Anschläge zu ziehen und so eine gute Fixierung des inneren Rings in seiner Position zu erzielen. Dafür stehen sich erste und zweite Magnete mit unterschiedlichen Polen gegenüber. Da der äußere Ring weitergedreht werden kann, als der innere Ring folgen kann, üben die Magnete die gewisse Kraft aus, um den inneren Ring gegen einen der ersten Anschläge zu ziehen und so zu fixieren.

Bei einer weiteren bevorzugten Ausgestaltung weist der zweite Vorsprung eine Raste auf, die mit einer Feder nach innen gegen das Gehäuse gedrückt wird, und wirkt die Raste mit mindestens einer Ausnehmung am Gehäuse zusammen, um den äußeren Ring in mindestens einer definierten Position lösbar zu fixieren.

Diese Ausgestaltung ermöglicht es, den äußeren Ring an mindestens eines definierten Position zu fixieren. Zudem kann durch das Einrasten der Raste in die Ausnehmung eine haptische Rückmeldung erzielt werden.

Bei einer weiteren bevorzugten Ausgestaltung ist die Lichtzuführung und/oder die Lichtführung ein Lichtwellenleiter.

Es versteht sich, dass die oben und im Folgenden beschriebenen Merkmale nicht nur in den ausdrücklich genannten Kombinationen, sondern auch in anderen Kombinationen und auch isoliert verwendet werden können.

Ausführungsbeispiele der Erfindung sind in der Zeichnung dargestellt und werden in der nachfolgenden Beschreibung näher erläutert. Es zeigen:
- Figur 1: eine Ausführungsform eines Endoskops in einer ersten perspektivischen Ansicht;
- Figur 2: die Ausführungsform in einer zweiten perspektivischen Ansicht;
- Figur 3: die Ausführungsform ohne ein Gehäuse mit Ringen in einer zweiten Position in einer ersten perspektivischen Ansicht;
- Figur 4: die Ausführungsform ohne ein Gehäuse in einer zweiten perspektivischen Ansicht;
- Figur 5: die Ansicht aus Figur 3 in teilweiser Explosionsdarstellung;
- Figur 6: die Ansicht aus Figur 5 als Drahtmodell;
- Figur 7: einen Schnitt durch das proximale Ende der Ausführungsform;
- Figur 8: die Ausführungsform ohne das Gehäuse mit den Ringen in einer Zwischenposition;
- Figur 9: die Ausführungsform ohne das Gehäuse mit den Ringen in einer ersten Position;
- Figur 10: eine schematische Darstellung der Ringe der Ausführungsform im Zusammenspiel mit dem Gehäuse;
- Figur 11: eine schematische Darstellung der Funktionsweise einer Hülse bei der Ausführungsform;
- Figur 12: eine Schnittdarstellung der Ausführungsform entlang der Längsachse mit den Ringen in der zweiten Position;
- Figur 13: eine Draufsicht auf die Ringe in der zweiten Position;
- Figur **14**: eine Schnittdarstellung der Ausführungsform entlang der Längsachse mit den Ringen in der ersten Position; und
- Figur 15: eine Draufsicht auf die Ringe in der ersten Position.

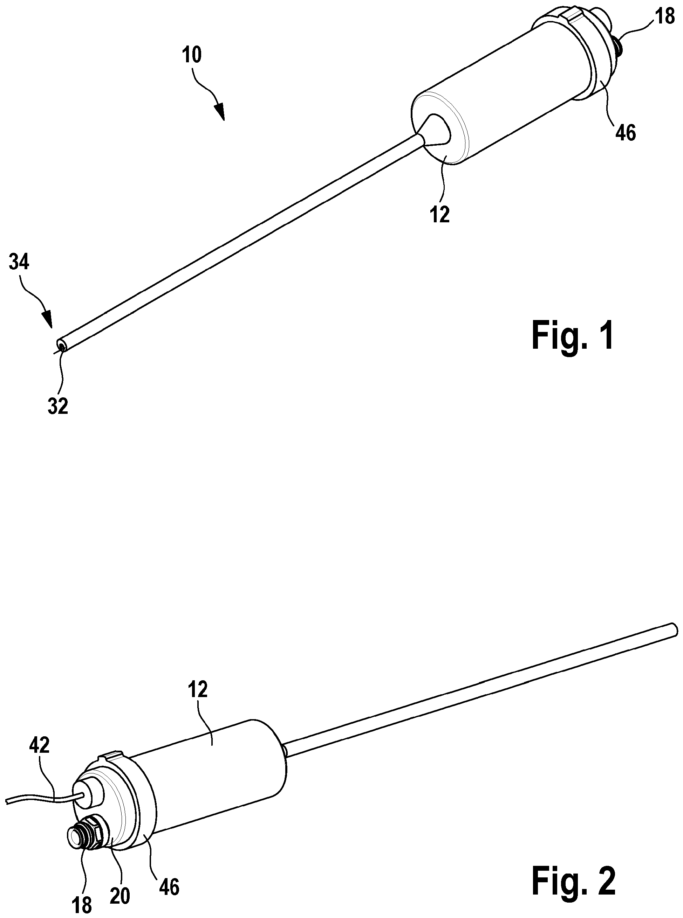

Die Figur 1 zeigt, in Verbindung mit den Figuren 2 bis 5, ein Endoskop 10, als Beispiel für ein optisches Instrument, mit einem Gehäuse 12, wobei in dem Gehäuse 12 eine Lichtquelle 14 angeordnet ist. Die Lichtquelle 14 ist dafür ausgebildet, einen ersten Lichtstrom zu erzeugen, der an einer ersten Grenzfläche 16 innerhalb des Gehäuses 12 austritt. Ein Exoskop stellt ein weiteres Beispiel für ein optisches Instrument dar.

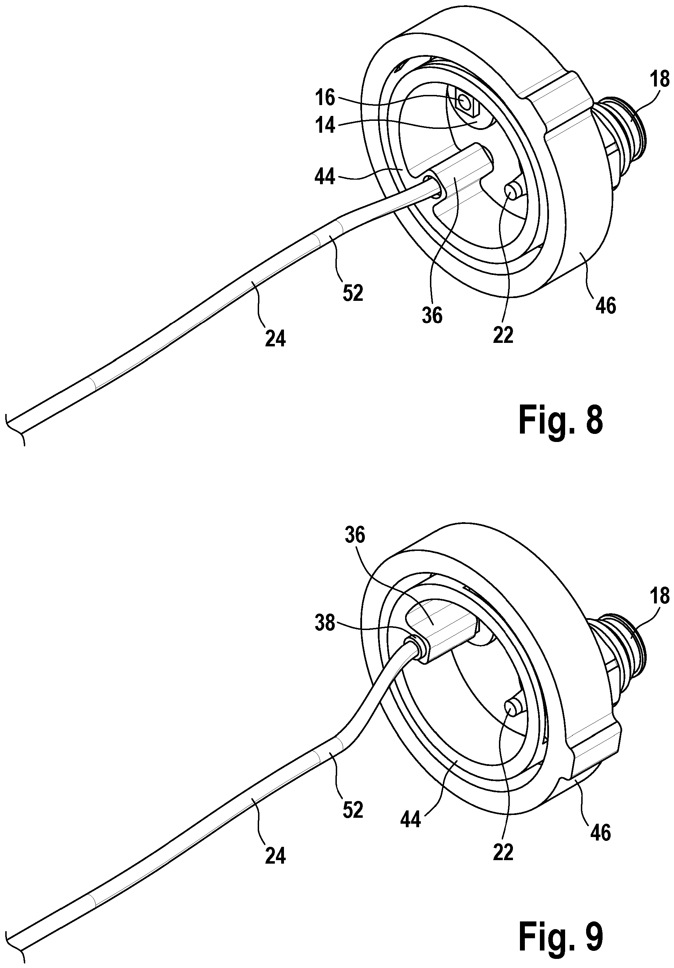

Eine Lichtzuführung 18 ist von außerhalb des Gehäuses 12 durch eine Gehäusewand 20 des Gehäuses 12 geführt. Die Lichtzuführung 18 ist dafür ausgebildet, einen zweiten Lichtstrom von außerhalb des Gehäuses 12 in ein Inneres des Gehäuses 12 zu führen und an einer zweiten Grenzfläche 22 (siehe Figur 8) innerhalb des Gehäuses 12 austreten zu lassen.

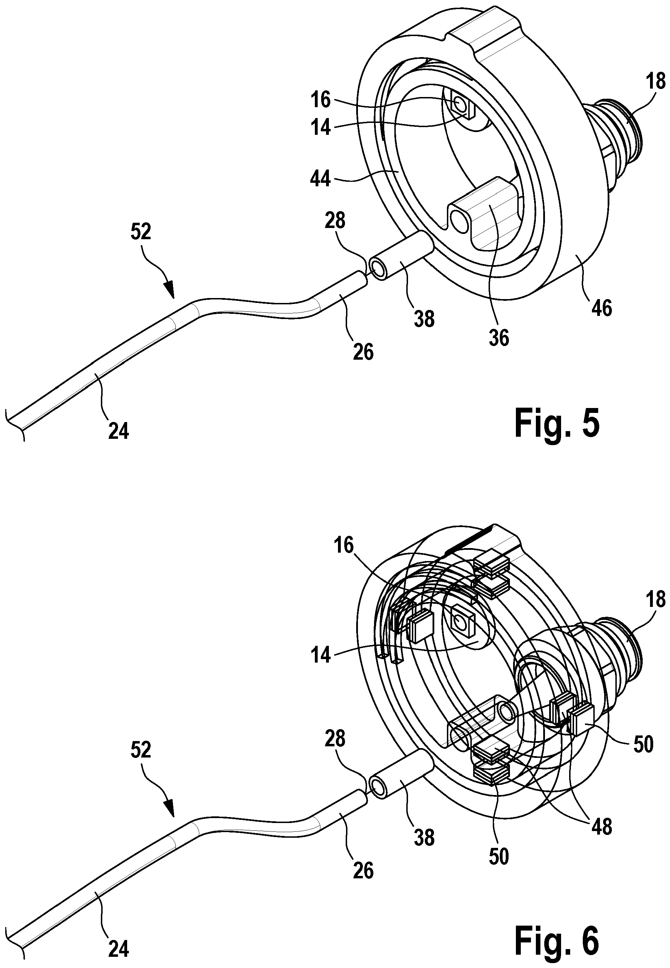

In dem Gehäuse 12 ist eine Lichtführung 24 angeordnet, die dafür ausgebildet ist, Licht an einem ersten Ende 26 über eine dritte Grenzfläche 28 (siehe Figur 5) aufzunehmen und an einem zweiten Ende 30 über eine vierte Grenzfläche 32 an einem distalen Ende 34 des Endoskops 10 austreten zu lassen.

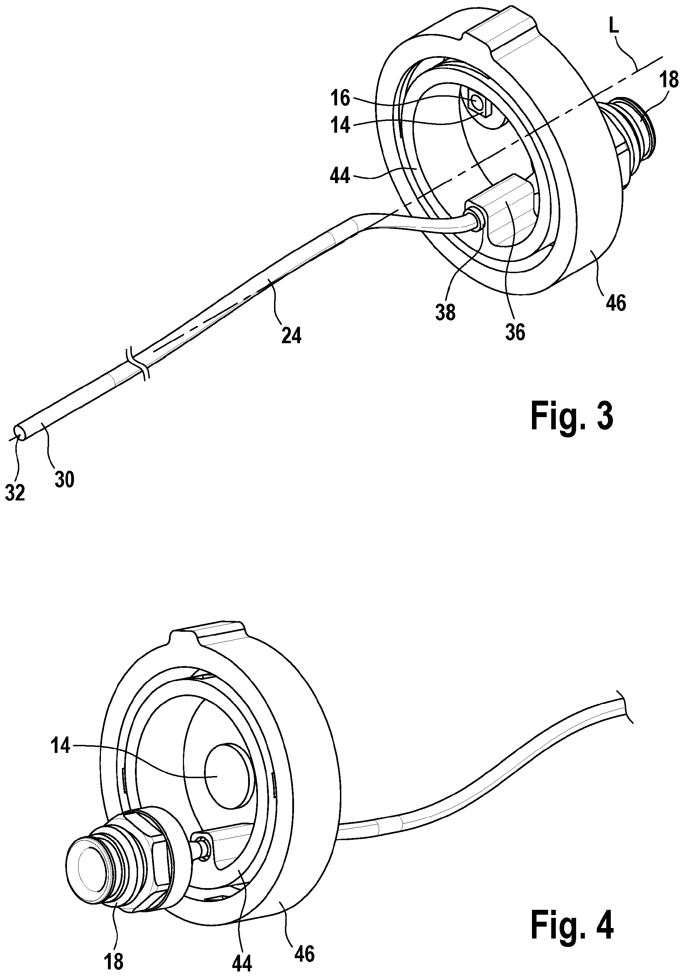

Die dritte Grenzfläche 28 ist relativ zu der ersten und der zweiten Grenzfläche 16, 22 derart verlagerbar angeordnet ist, dass die dritte Grenzfläche 28 in einer ersten Position (siehe Figur 9) Licht von der ersten Grenzfläche 16 aufnehmen kann und in einer zweiten Position (siehe Figur 3) Licht von der zweiten Grenzfläche 22 aufnehmen kann.

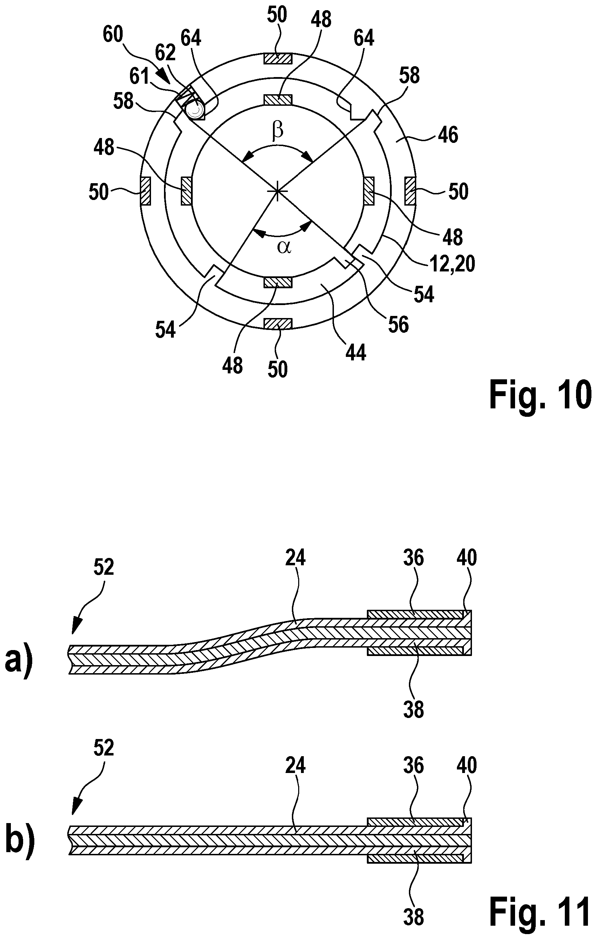

Das erste Ende 26 ist in einer Führung 36 linear verlagerbar gehalten. Dafür weist das erste Ende 26 eine Hülse 38 auf und die Hülse 38 ist in der Führung 36 linear verlagerbar gehalten. Die Führung 36 und die Hülse 38 sind hier zylinderförmig ausgebildet. Zudem kann, siehe Figur 11, die Hülse 38 einen Flansch 40 aufweisen, der einen Anschlag für eine Verlagerung der Hülse 38 bildet, hier einen Anschlag in distaler Richtung.

Wie in der Figur 2 angedeutet ist, wird die Lichtquelle 14 über eine Energiezuführung 42, die von außerhalb des Gehäuses 12 durch die Gehäusewand 20 des Gehäuses 12 geführt ist, mit Energie versorgt.

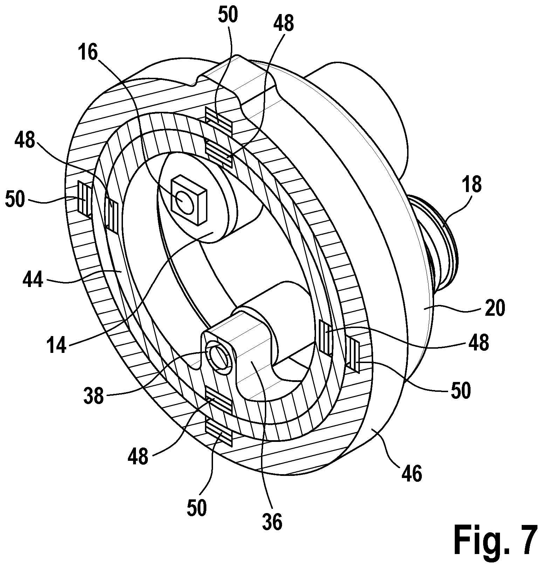

Ausgehend von den Figuren 5, 6 und 7 wird nun erläutert, wie das Umschalten zwischen der eine Lichtquelle 14 und der Lichtzuführung 18 erfolgen kann. Das erste Ende 26 ist auf einem inneren Ring 44 innerhalb des Gehäuses 12 angeordnet, der um eine Längsachse L des Endoskops 10 rotierbar angeordnet ist. Dabei kann die Längsachse L des Endoskops 10 bei einigen Ausgestaltungen die Längsmittelachse des Endoskops 10 sein.

Das Endoskop 10 weist ferner einen äußeren Ring 46 auf, der außerhalb des Gehäuses 12 angeordnet ist und um die Längsachse L des Endoskops 10 rotierbar angeordnet ist. Am inneren Ring 44 ist mindestens ein erster Magnet 48 angeordnet, und am äußeren Ring 46 ist mindestens ein zweiter Magnet 50 angeordnet. Hier sind jeweils vier Magnete 48, 50 vorhanden. Die ersten Magnete 48 und die zweiten Magnete 50 wirken derart zusammen, dass mittels einer Rotation des äußeren Rings 46 eine Rotation des inneren Rings 44 bewirkt werden kann. Dabei kann, wie hier gezeigt, die anziehende Kraft unterschiedlicher Pole genutzt werden, alternativ aber auch die abstoßende Kraft gleicher Pole.

Die Figuren 8 und 9 zeigen, wie das magnetische Zusammenwirken der ersten Magnete 48 und die zweiten Magnete 50 bei einer Rotation des äußeren Rings 46 zu einer entsprechenden Verlagerung des inneren Rings 44 und damit des Endes 26 führt. So kann trotz des Gehäuses 12, welches sich zwischen dem inneren Ring 44 und dem äußeren Ring 46 befindet, eine Bedienung erfolgen, ohne den hermetischen Verschluss des Gehäuses 12 zu schwächen.

Es ist dabei in der Figur 8 zu erkennen, dass sich das Ende 26 mit der Hülse 38 in die proximale Richtung verlagert hat. Dies ermöglicht eine Verlagerung des Endes 26, so dass die Lichtführung 24 am Gehäuse an einem Fixpunkt 52 befestigt sein kann und dennoch ein Längenausgleich der Lichtführung 24 bei einer Drehung des inneren Rings 44 erfolgen kann.

Figur 10 zeigt, dass das Gehäuse 12 bzw. die Gehäusewand 20 zwei nach innen gerichtete erste Anschläge 54 aufweist. Die ersten Anschläge 54 wirken mit einem ersten Vorsprung 56 am inneren Ring 44 zusammen und begrenzen eine Verlagerung des inneren Rings 44 auf einen ersten Winkelbereich α.

Das Gehäuse 12 bzw. die Gehäusewand 20 weist ferner zwei nach außen gerichtete zweite Anschläge 58 auf, die mit einem zweiten Vorsprung 60 am äußeren Ring 46 zusammenwirken und eine Verlagerung des äußeren Rings 46 auf einen zweiten Winkelbereich β begrenzen, wobei der zweite Winkelbereich größer als der erste Winkelbereich ist.

Der zweite Vorsprung 60 weist eine Raste 62, die mit einer Feder 61 nach innen gegen das Gehäuse 12 gedrückt wird. Die Raste 62 wirkt mit mindestens einer Ausnehmung 64 am Gehäuse 12 zusammen, um den äußeren Ring 46 in mindestens einer definierten Position lösbar zu fixieren. Die Raste 62 ist hier als Kugelraste ausgeführt.

In Figur 11 sind zwei beispielhafte Situationen dargestellt, wie sie sich bei einer Rotation des inneren Rings 44 ergeben können. Dabei kann die Darstellung a) der Situation entsprechen, wie sie in Figur 3 oder Figur 9 gezeigt ist, und die Darstellung b) der Situation entsprechen, wie sie in Figur 8 gezeigt ist.

Bei der Darstellung a) ist die Strecke vom Fixpunkt 52 zum Eingang in die Führung 36 etwas größer als bei der Darstellung b). Der Flansch 40 liegt bei der Darstellung a) am proximalen Ende der Führung 36 an. Bei der Darstellung b) hat sich der Flansch 40 von der Hülse 36 gelöst. Dadurch kann der Verkürzung der Strecke Rechnung getragen werden und ein mechanischer Widerstand durch ein Stauchen bzw. Verbiegen der Lichtführung 24 vermieden werden.

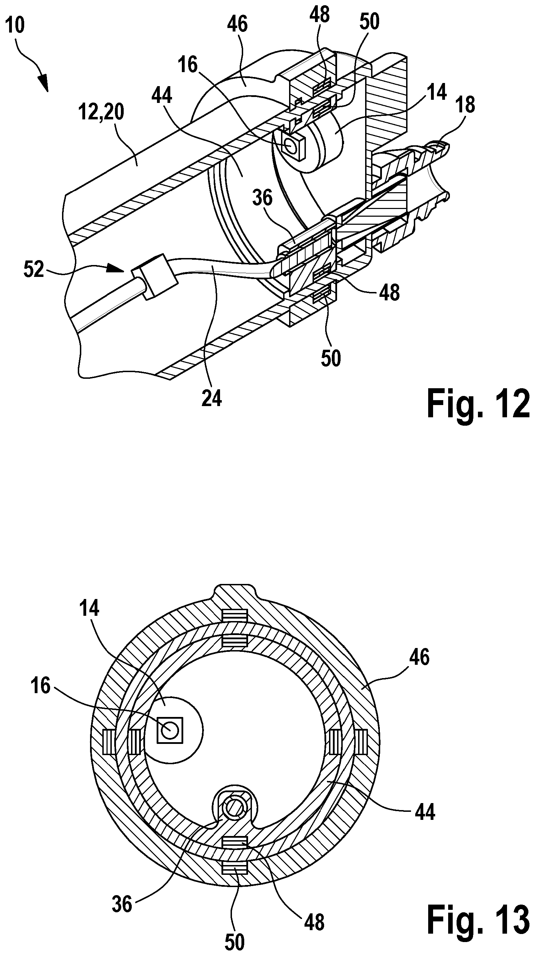

Figur 12 zeigt eine Schnittdarstellung der Ausführungsform entlang der Längsachse L mit den Ringen in der zweiten Position, und Figur 13 zeigt eine Draufsicht auf die Ringe in der zweiten Position. Die dritte Grenzfläche 28 ist hier ebenfalls in ihrer zweiten Position.

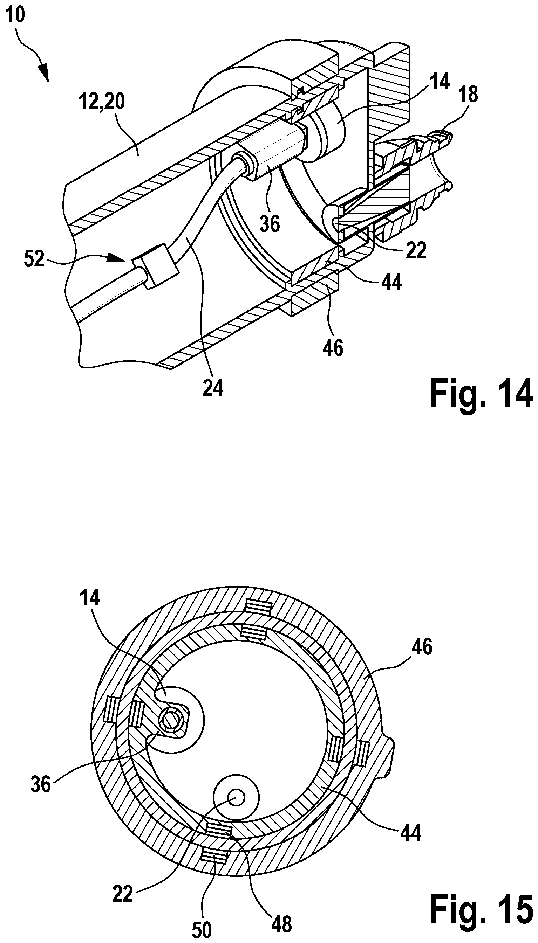

Figur 14 zeigt eine Schnittdarstellung der Ausführungsform entlang der Längsachse L mit den Ringen in der ersten Position, und Figur 15 zeigt eine Draufsicht auf die Ringe in der ersten Position. Die dritte Grenzfläche 28 ist hier ebenfalls in ihrer ersten Position.

## Patentansprüche

1. Optisches Instrument mit einem Gehäuse (12), wobei in dem Gehäuse (12) eine Lichtquelle (14) angeordnet ist, die dafür ausgebildet ist, einen ersten Lichtstrom zu erzeugen, der an einer ersten Grenzfläche (16) innerhalb des Gehäuses (12) austritt, und eine Lichtzuführung (18) von außerhalb des Gehäuses (12) durch eine Gehäusewand (20) des Gehäuses (12) geführt ist, die dafür ausgebildet ist, einen zweiten Lichtstrom von außerhalb des Gehäuses (12) in ein Inneres des Gehäuses (12) zu führen und an einer zweiten Grenzfläche (22) innerhalb des Gehäuses (12) austreten zu lassen, wobei in dem Gehäuse (12) eine Lichtführung (24) angeordnet ist, die dafür ausgebildet ist, Licht an einem ersten Ende (26) über eine dritte Grenzfläche (28) aufzunehmen und an einem zweiten Ende (30) über eine vierte Grenzfläche (32) an einem distalen Ende (34) des optischen Instruments austreten zu lassen, wobei die dritte Grenzfläche (28) relativ zu der ersten und der zweiten Grenzfläche (16, 22) derart verlagerbar angeordnet ist, dass die dritte Grenzfläche (28) in einer ersten Position Licht von der ersten Grenzfläche (16) aufnehmen kann und in einer zweiten Position Licht von der zweiten Grenzfläche (22) aufnehmen kann.

2. Optisches Instrument nach Anspruch 1, wobei das erste Ende (26) der Lichtführung (24) in einer Führung (36) linear verlagerbar gehalten ist.

3. Optisches Instrument nach Anspruch 2, wobei das erste Ende (26) eine Hülse (38) aufweist und die Hülse (38) in der Führung (36) linear verlagerbar gehalten ist.

4. Optisches Instrument Anspruch 2 oder 3, wobei die Führung (36) und die Hülse (38) zylinderförmig ausgebildet sind.

5. Optisches Instrument nach Anspruch **4,** wobei die Hülse (38) einen Flansch (40) aufweist, der einen Anschlag für eine Verlagerung der Hülse (38) bildet.

6. Optisches Instrument nach einem der vorhergehenden Ansprüche, wobei die Lichtquelle (14) über eine Energiezuführung (42), die von außerhalb des Gehäuses (12) durch die Gehäusewand (20) des Gehäuses (12) geführt ist, mit Energie versorgt ist.

7. Optisches Instrument nach einem der vorhergehenden Ansprüche, wobei das erste Ende (26) auf einem inneren Ring (44) innerhalb des Gehäuses (12) angeordnet ist, der um eine Längsachse (L) des optischen Instruments rotierbar angeordnet ist.

8. Optisches Instrument nach Anspruch 7, ferner mit einem äußeren Ring (46), der außerhalb des Gehäuses (12) angeordnet ist und um die Längsachse (L) des optischen Instruments rotierbar angeordnet ist, wobei am inneren Ring (44) mindestens ein erster Magnet (48) angeordnet ist, am äußeren Ring (46) mindestens ein zweiter Magnet (50) angeordnet ist und der erste Magnet (48) und der zweite Magnet (50) derart zusammenwirken, dass mittels einer Rotation des äußeren Rings (46) eine Rotation des inneren Rings (44) bewirkt werden kann.

9. Optisches Instrument nach Anspruch 8, wobei das Gehäuse (12) zwei nach innen gerichtete erste Anschläge (54) aufweist, die mit einem ersten Vorsprung (56) am inneren Ring (44) zusammenwirken und eine Verlagerung des inneren Rings (44) auf einen ersten Winkelbereich (α) begrenzen, und zwei nach außen gerichtete zweite Anschläge (58) aufweist, die mit einem zweiten Vorsprung (60) am äußeren Ring (46) zusammenwirken und eine Verlagerung des äußeren Rings auf einen zweiten Winkelbereich (β) begrenzen, wobei der zweite Winkelbereich (β) größer als der erste Winkelbereich (α) ist.

10. Optisches Instrument nach Anspruch 9, wobei der zweite Vorsprung (60) eine Raste (62) aufweist, die mit einer Feder (61) nach innen gegen das Gehäuse (12) gedrückt wird und die Raste (62) mit mindestens einer Ausnehmung am Gehäuse (64) zusammenwirkt, um den äußeren Ring (46) in mindestens einer definierten Position lösbar zu fixieren.

11. Optisches Instrument nach einem der vorhergehenden Ansprüche, wobei die Lichtzuführung (18) und/oder die Lichtführung (24) einen Lichtwellenleiter aufweist.

## Claims

1. An optical instrument with a housing (12), wherein a light source (14) is arranged in the housing (12) and is designed to generate a first light flux which emerges at a first boundary surface (16) inside the housing (12), and a light supply (18) is guided from outside the housing (12) through a housing wall (20) of the housing (12), which is designed to guide a second light flux from outside the housing (12) into an interior of the housing (12) and to allow it to emerge at a second boundary surface (22) inside the housing (12), wherein a light guide (24) is arranged in the housing (12) and is designed to receive light at a first end (26) via a third boundary surface (28) and to allow it to emerge at a second end (30) via a fourth boundary surface (32) at a distal end (34) of the optical instrument, wherein the third boundary surface (28) is arranged to be displaceable relative to the first and second boundary surfaces (16, 22) in such manner that the third boundary surface (28) can receive light from the first boundary surface (16) in a first position and can receive light from the second boundary surface (22) in a second position.

2. The optical instrument according to claim 1, wherein the first end (26) of the light guide (24) is held linearly displaceably in a guide (36).

3. The optical instrument according to claim 2, wherein the first end (26) has a sleeve (38) and the sleeve (38) is held linearly displaceably in the guide (36).

4. The optical instrument claim 2 or 3, wherein the guide (36) and the sleeve (38) are cylindrical.

5. The optical instrument according to claim 4, wherein the sleeve (38) has a flange (40) which forms a stop for a displacement of the sleeve (38).

6. The optical instrument according to one of the preceding claims, wherein the light source (14) is supplied with power via a power supply (42) which is guided from outside the housing (12) through the housing wall (20) of the housing (12).

7. The optical instrument according to one of the preceding claims, wherein the first end (26) is arranged on an inner ring (44) inside the housing (12), which is arranged so as to be rotatable about a longitudinal axis (L) of the optical instrument.

8. The optical instrument according to claim 7, further comprising an outer ring (46) which is arranged outside the housing (12) and is arranged so as to be rotatable about the longitudinal axis (L) of the optical instrument, wherein at least one first magnet (48) is arranged on the inner ring (44), at least one second magnet (50) is arranged on the outer ring (46) and the first magnet (48) and the second magnet (50) cooperate in such manner that a rotation of the inner ring (44) can be effected by means of a rotation of the outer ring (46).

9. The optical instrument according to claim 8, wherein the housing (12) has two inwardly directed first stops (54) which cooperate with a first projection (56) on the inner ring (44) and limit a displacement of the inner ring (44) to a first angular range (α), and has two outwardly directed second stops (58) which cooperate with a second projection (60) on the outer ring (46) and limit a displacement of the outer ring to a second angular range (β), wherein the second angular range (β) is greater than the first angular range (α).

10. The optical instrument according to claim 9, wherein the second projection (60) has a catch (62) which is pressed inwards against the housing (12) by a spring (61) and the catch (62) cooperates with at least one recess on the housing (64) in order to detachably fix the outer ring (46) in at least one defined position.

11. The optical instrument according to one of the preceding claims, wherein the light supply (18) and/or the light guide (24) has an optical waveguide.

## Revendications

1. Instrument optique comportant un boîtier (12), dans lequel une source de lumière (14) est agencée dans le boîtier (12), qui est réalisée pour produire un premier flux lumineux, qui sort par une première interface (16) à l'intérieur du boîtier (12), et une alimentation en lumière (18) est guidée depuis l'extérieur du boîtier (12) à travers une paroi de boîtier (20) du boîtier (12), qui est réalisée pour guider un second flux lumineux depuis l'extérieur du boîtier (12) dans un intérieur du boîtier (12) et le laisser sortir par une deuxième interface (22) à l'intérieur du boîtier (12), dans lequel un guide de lumière (24) est agencé dans le boîtier (12), qui est réalisé pour recevoir de la lumière à une première extrémité (26) par l'intermédiaire d'une troisième interface (28) et la laisser sortir à une seconde extrémité (30) par l'intermédiaire d'une quatrième interface (32) à une extrémité distale (34) de l'instrument optique, dans lequel la troisième interface (28) est agencée de manière à pouvoir être déplacée par rapport à la première et à la deuxième interface (16, 22) de telle sorte que la troisième interface (28) peut recevoir de la lumière provenant de la première interface (16) dans une première position et peut recevoir de la lumière provenant de la deuxième interface (22) dans une seconde position.

2. Instrument optique selon la revendication 1, dans lequel la première extrémité (26) du guide de lumière (24) est maintenue de manière à pouvoir être déplacée linéairement dans un guide (36).

3. Instrument optique selon la revendication 2, dans lequel la première extrémité (26) présente une douille (38) et la douille (38) est maintenue de manière à pouvoir être déplacée linéairement dans le guide (36).

4. Instrument optique revendication 2 ou 3, dans lequel le guide (36) et la douille (38) sont réalisés de forme cylindrique.

5. Instrument optique selon la revendication 4, dans lequel la douille (38) présente une bride (40), qui forme une butée pour un déplacement de la douille (38).

6. Instrument optique selon l'une des revendications précédentes, dans lequel la source de lumière (14) est approvisionnée par l'intermédiaire d'une alimentation en énergie (42), qui est guidée depuis l'extérieur du boîtier (12) à travers la paroi de boîtier (20) du boîtier (12), en énergie.

7. Instrument optique selon l'une des revendications précédentes, dans lequel la première extrémité (26) est agencée sur une bague intérieure (44) à l'intérieur du boîtier (12), qui est agencée de manière à pouvoir tourner autour d'un axe longitudinal (L) de l'instrument optique.

8. Instrument optique selon la revendication 7, comportant en outre une bague extérieure (46), qui est agencée à l'extérieur du boîtier (12) et qui est agencée de manière à pouvoir tourner autour de l'axe longitudinal (L) de l'instrument optique, dans lequel au moins un premier aimant (48) est agencé sur la bague intérieure (44), au moins un second aimant (50) est agencé sur la bague extérieure (46) et le premier aimant (48) et le second aimant (50) coopèrent de telle sorte qu'au moyen d'une rotation de la bague extérieure (46), une rotation de la bague intérieure (44) peut être provoquée.

9. Instrument optique selon la revendication 8, dans lequel le boîtier (12) présente deux premières butées (54) dirigées vers l'intérieur, qui coopèrent avec une première saillie (56) sur la bague intérieure (44) et limitent un déplacement de la bague intérieure (44) à une première plage angulaire (α), et présente deux secondes butées (58) orientées vers l'extérieur, qui coopèrent avec une seconde saillie (60) sur la bague extérieure (46) et limitent un déplacement de la bague extérieure à une seconde plage angulaire (β), dans lequel la seconde plage angulaire (β) est supérieure à la première plage angulaire (α).

10. Instrument optique selon la revendication 9, dans lequel la seconde saillie (60) présente un cran (62), qui est pressé vers l'intérieur contre le boîtier (12) par un ressort (61) et le cran (62) coopère avec au moins un évidement sur le boîtier (64), pour fixer de manière amovible la bague extérieure (46) dans au moins une position définie.

11. Instrument optique selon l'une des revendications précédentes, dans lequel l'alimentation en lumière (18) et/ou le guide de lumière (24) présentent une fibre optique.
